# EUROPEAN PATENT SPECIFICATION

(11) **EP 3 413 974 B1**
(45) Date of publication and mention of the grant of the patent: **23.08.2023**
(21) Application number: 17749990.2
(22) Date of filing: 12.02.2017
(51) Int. Cl.: A61N 5/06, A61N 5/067, A61B 18/20, A61N 5/02, A61B 18/00, A61N 7/02, A61N 5/00, A61N 7/00, A61B 18/18, A61N 1/40

(54) **APPARATUS AND COSMETIC METHOD FOR TREATING HYPERHIDROSIS**
VORRICHTUNG UND KOSMETISCHES VERFAHREN ZUR BEHANDLUNG VON HYPERHIDROSE
DISPOSITIF ET PROCÉDÉ COSMÉTIQUE DE TRAITEMENT DE L'HYPERHIDROSE

(30) Priority: 13.02.2016 US 201662295032 P
(43) Date of publication of application: 19.12.2018
(73) Proprietor: Lumenis Be Ltd., 20692 Yokneam Illit (IL)
(72) Inventor: GREENBAUM, Lior, 42383 Kadima Tzoran (IL); SCHUSTER, Israel, 36090 Kiryat Tivon (IL); KOIFMAN, Danny, 55900 Gena Tikva (IL)
(74) Representative: Stütz, Jan
(86) International application number: PCT/IL2017/050174
(87) International publication number: WO 2017/138001

(56) References cited:
- WO-A1-2009/147617
- WO-A2-03/001984
- US-A1- 2005 010 271
- US-A1- 2010 174 222
- US-A1- 2011 190 745
- US-A1- 2014 207 213

## Description

### FIELD OF THE INVENTION

The present invention relates to an apparatus and a cosmetic method for treating hyperhidrosis with light-based treatments.

### BACKGROUND OF THE PRESENT INVENTION

### Photothermal Treatment

The treatment of the human body with energy sources (such as IPL, laser, Ultrasound or RF) usually induces an increase in the temperature of selected tissues, causing cellular proteins to denature, aggregate, and lose their biological activity. This in turn leads eventually to cell necrosis and/or apoptosis. The complexity of metabolic and regulatory pathways used by living cells leads to complex mechanisms during the necrotic and/or apoptotic process as well. Some studies conclude that the lipid bilayer, and perhaps some membrane-bound ATPase enzymes, are the macromolecules most likely implicated in thermal damage. (Despa, F., Orgill, D.P., Neuwalder, J., Lee, R.C., "The relative thermal stability of tissue macromolecules and cellular structure in burn injury," Burns 31(5), 568-577 (2005)).

In general, it is the combination of temperature levels and the exposure time to elevated temperatures that induces necrotic processes in living cells. The term "accumulated thermal dose" may be used to describe this combination. Several studies indicate that there is a correlation between cell death and the rate of delivery of accumulated thermal dose during photothermal exposure. (Michael L. Denton; Gary D. Noojin; B. Giovanna Gamboa; Elharith M. Ahmed; Benjamin A. Rockwell, "Photothermal damage is correlated to the delivery rate of time-integrated temperature", SPIE 9706, Optical Interactions with Tissue and Cells XXVII, 97061M (7 March 2016).

When targeting specific organs (e.g. sweat glands, sebaceous glands, hair follicles...), the objective is to induce the tissue destruction process only in the targeted organ, while leaving the surrounding tissue intact, namely, increasing the target organ temperature to levels where necrotic processes are initiated, while maintaining surrounding tissue temperature at safe levels. There are several approaches that can be taken to accomplish that objective.

One approach is to utilize differences in absorption spectra and use of one or more specific light wave bands that are better absorbed by the targeted organ than its surrounding tissue.

Inducing the desired temperature difference can also be obtained by using the energy source in a pulsed mode. When designed properly, differences in thermal relaxation times (TRT) cause the target tissue to maintain its elevated temperature for longer time, increasing the probability of necrotic processes to occur.

Bulk heating using a continuous wave-type laser or other source can also be utilized in some cases. As mentioned above, necrotic and/or apoptosis processes are complex and different organs will require different temperature levels to initiate these processes. If a tissue damage process occurs in a specific organ at lower temperature than in the surrounding tissue, heating the tissue bulk to that temperature for long enough will eventually induce necrotic process to the target organs with potentially minimal effect on the surrounding tissue. This is likely what occurs when utilizing bulk heating for hair removal.

Document US 2010/174222 discusses hyperhidrosis treatment wherein wavelength of 980 nm may be used. The tissue is exposed to about 2 to about 2000 pulses, wherein each pulse is from about 1 millisecond to about 1000 milliseconds and the pulses are separated by from about 1 to about 1000 milliseconds. Energy fluences may range from about 0.01 J/cm2 to about 20 J/cm².

### Sweat Glands

Sweating occurs in all humans. It is the body's way of ridding itself of wastes as well as cooling off the body. In many people, excessive, bothersome sweating is not a problem given the availability of deodorants and the availability of anti-perspirants to basically block excretion of sweat. For other people, however, sweating may be a major problem or present an embarrassing situation not resolvable by topical lotions.

Sweat glands are cutaneous appendages like hair follicles or sebaceous glands

Sweat glands are coiled tubular systems and generally fall under two types: eccrine sweat glands and apocrine sweat glands, as will be described below. Although they are usually perceived as distinct single entities, there is evidence that the coils of two eccrine glands may also occur convoluted within each other; thus, these structures might easily be mistaken as one large coil with two or more ducts to the surface.

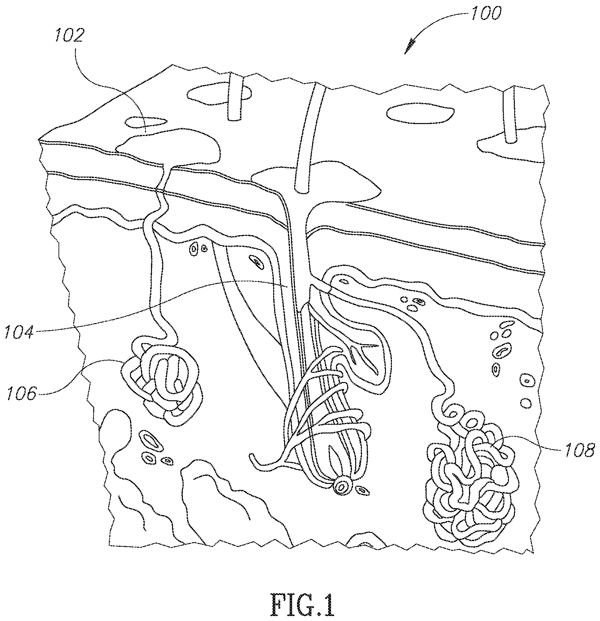

There is a distinction between eccrine and apocrine glands, which differ one from the other in their morphology and content (sweat ingredients). Figure 1 illustrates a schematic rendering of these glands. In Figure 1, reference numeral 100 represents the surface of the skin tissue. The sweat pore 102 is shown as reaching the surface of the skin tissue so that secretions can be released to the outside of the skin tissue. An eccrine-type of sweat gland 106 is shown as is an apocrine-type sweat gland 108 which surrounds a hair follicle shaft 104. Typically, eccrine glands occur over most of the body and open directly onto the surface of the skin, while apocrine sweat glands open into a hair follicle, as illustrated, leading to the surface of the skin. Apocrine glands develop in areas abundant in hair follicles, such as the scalp, armpits and groin areas.

The eccrine gland consists of a single tubule ranging from about 4 to 8 mm in total length. The intra-epidermal part of the sweat gland is called the acrosyringium followed by the dermal duct with a straight and a coiled part, and a coiled, secretory portion, which is found in the deep dermis, as can be seen in Figure 1. 99% of the eccrine sweat content is water, the remainder being salts and proteins.

Apocrine glands are generally restricted to hairy body areas, as they open and secrete into the hair canal. For this reason, apocrine glands can be found only in the axilla, mammary, perineal and genital region. The apocrine duct is very short and can be found in close vicinity to the hair follicle, again as may be seen in Figure 1.

Some human sweat glands cannot be classified as either apocrine or eccrine, having characteristics of both; such glands are termed apoeccrine. (See, Wikipedia description of "Sweat Gland" at wikipedia.org.)

Apoeccrine glands are larger than eccrine glands, but smaller than apocrine ones; their secretory portion has a narrow portion similar to the secretory coils in eccrine glands as well as a wide section reminiscent of apocrine glands. Apoeccrine glands, found in the axilla and perianal region, have ducts opening onto the skin surface. They are presumed to have developed in puberty from the eccrine glands, and can comprise up to 50% of all axillary glands. Apoeccrine glands secrete more sweat than both eccrine and apocrine glands, thus playing a large role in axillary sweating.

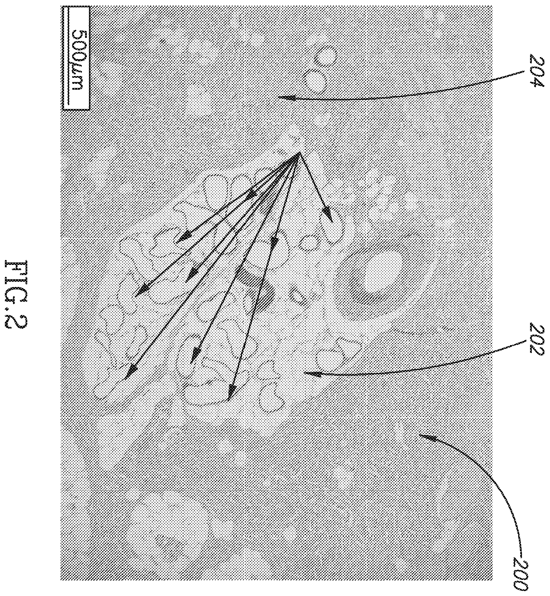

Sweat glands are found, at least in part, to be located within fat islands assimilated in the dermal layer, as well as in the dermis-hypodermis boundary and in the upper layer of hypodermis. Thus, a substantial portion of the sweat glands is surrounded by a fat layer. Figure 2 illustrates this relationship between fat cells and sweat glands, in which the dermis 200 is there shown to include sweat glands 204 found within a fat pocket 202 in the dermis.

### Hyperhidrosis

There are several methods by which heavy sweating (Hyperhidrosis) may be treated. The easiest and probably most commonly used way is to apply an antiperspirant (many of which contain aluminum salts) to the skin tissue surface, which forms a "plug" that blocks excretions in the form of perspiration. Many people use such antiperspirant on a daily basis.

In cases where antiperspirants do not solve an excess sweating problem, there are several medical treatments that may remedy such excessive sweating:
Iontophoresis: During this treatment, a low electrical current travels through shallow water in which the patient immerses his hands or feet (or both). Each treatment takes about 20 to 30 minutes and the patient has to repeat it at least a few times a week. No one knows exactly how this treatment works, but experts believe it blocks sweat from getting to the skin surface.

Botulinum toxin: Another treatment option for heavy sweating is injections of botulinum toxin A (Botox). It is a natural, purified protein which, when small doses are injected into the skin, blocks the nerves that supply the eccrine glands; this prevents the glands from producing sweat. By blocking, or interrupting, this chemical messenger, botulinum toxin "turns off" sweating at the area where it has been injected. Botox injections are very shallow, meaning that the materials are injected just below the surface of the skin, where they remain. Botox is FDA-approved for treating excessive sweating of the underarms, and it has been shown to result in an 82-87% decrease in sweating. Some doctors may also use Botox on the palms of the hands and soles of the feet, where it presents 80% -90% efficacy. The treatment is not a cure for hyperhidrosis - it only provides temporary relief. It needs to be repeated every three to six months for maximum effect.

Anticholinergic drugs: There are oral anticholinergic drugs that stop the activation of the sweat glands. Due to the severe side effects such as blurred vision, heart palpitations, and urinary problems, they are usually not recommended.

Surgery: This may be the only option for those severe hyperhidrosis cases that have not responded to other treatments. During surgery, the doctor may cut, scrape, or suction out the sweat glands. One surgical option is endoscopic thoracic sympathectomy (ETS), in which the surgeon makes very small incisions and cuts the nerves in the armpit that normally activate the sweat glands. This procedure is very effective, but it's used only as a last resort on people who have tried every other treatment. ETS can't be reversed, and it can leave scars. One side effect almost everyone who receives an ETS treatment has to deal with is compensatory sweating. When the body stops sweating in one area, it may start sweating in another (such as the face or chest) to compensate.

Laser systems for minimally invasive solutions for hyperhidrosis are offered by several vendors such as the companies Fotona (XP2), Deka (Smartlipo) and Cynosure (SideLaze). The systems sold by these companies use laser light to permanently destroy axillary sweat glands by photo-thermally heating sweat gland tissue with minimal damage to the surrounding tissue. In some cases the laser is introduced into the subcutaneous tissue by an optical fiber. In these cases, a local anesthetic may be infused into the area to be treated, such as the armpits, before treatment. The same vendors claim to use a specific wavelength which is claimed to be selectively absorbed by the subcutaneous tissue of the armpit. The foregoing are offered by Fontona and Deka, which provide devices operating at 1064nm, and Cynosure, which provides a device operating at 1440nm. A company named Miramar Labs uses a non-invasive handheld device (miraDry) to deliver precisely controlled microwave energy beneath the underarm skin to the specific area in which sweat glands are located, resulting in thermolysis (decomposition by heat) of the sweat glands. During this treatment, the top layers of the skin are simultaneously cooled and protected. Sweat glands are not believed to grow back after treatment so the effect can be seen almost immediately and results may be lasting. Some patients may only need one treatment but, for best results, two procedures spaced three months apart may be recommended.

It is to the reduction of sweating by disrupting the excretions of sweat glands that this invention is directed.

### SUMMARY OF THE PRESENT INVENTION

The reduction of sweating by disrupting the excretion of sweat glands according to this invention can be achieved by using an apparatus according to claim 1 or performing a method according to claim 2.

In an aspect, a cosmetic method of treating a human body to reduce hyperhidrosis includes applying laser light energy to one or more portions of the human body in which the laser light energy is about 970nm. The laser light energy is delivered pulsed. A controller controls the laser light source to operate at the following parameters:
(a) fluence of 5-50 j/cm2;
(b) pulse width of 10-60ms;
(c) number of pulses of 1-20;
(d) repetition rate of 0.3 - 5Hz.

The pulse rate may be either 1 Hz or may be selected from between ½ Hz and 3 Hz.

In a further aspect, the methods may include the step of cooling the human body near the application of the laser light energy.

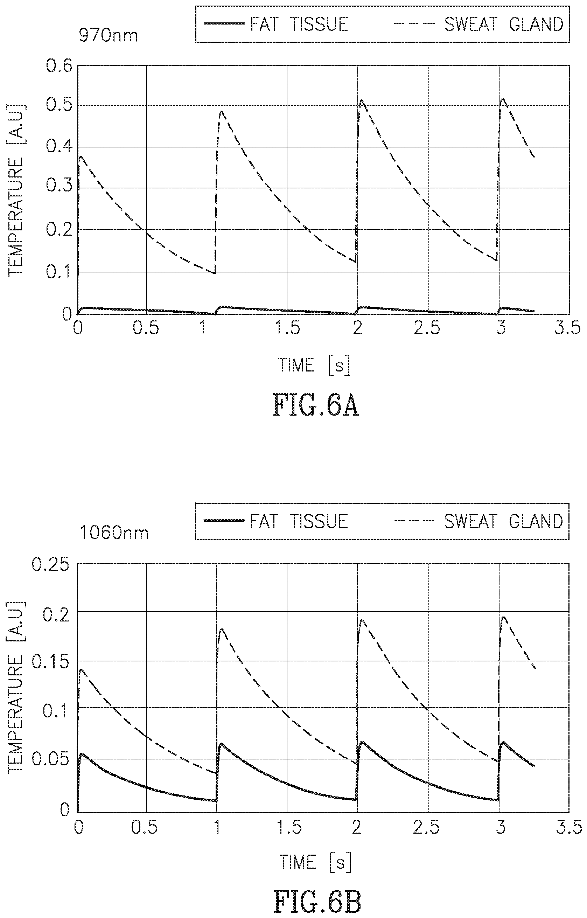

In another aspect, the time delay between pulses may be between the TRT of fat cells and the TRT of sweat glands. Thus, in Figs. 6A and 6B, the temperature of the sweat glands is seen to increase in temperature to a much higher level than those of the fatty components to cause selective damage of the sweat glands. It may also be possible to use different pulses, for example, pulses at a higher level in the beginning of the treatment to increase the temperature of the sweat gland cells to a target temperature or at least approaching the target temperature and then lowering the pulses to a lower level to maintain a steady temperature in the sweat cells to allow controlled development of the biochemical process in the sweat glands that eventually causes sweat gland damage.

In yet another aspect, the cosmetic method may further include the step of providing bulk heating of the skin surface of the human body. Bulk heating may be delivered as the main treatment modality, as will be described below and may also be delivered in conjunction with selective targeting treatment. When bulk heating is delivered in conjunction with selective targeting treatment it may be done in one or more of: before, during and after the application of selective targeting treatment. The bulk heating may be provided by one or more of: laser light energy, IPL light energy, RF energy, microwave energy or ultrasonic energy. The bulk heating may be applied at about 400nm to about 650nm.

In yet a further aspect, the cosmetic method may further include providing a handpiece to deliver the laser light energy; the handpiece may include an open tip and a source of vacuum, and may include the step of drawing the skin tissue into the open tip prior to applying the laser light energy, such that the sweat glands may be drawn closer to the laser light source. Such a handpiece, such as that handpiece illustrated in Figure 5, as described in US 2008/0119830 assigned to the same assignee of the present invention.

In yet another aspect, an apparatus for treating a human body to reduce hyperhidrosis includes a source of laser light energy; a controller for controlling the laser light source; the controller causing the laser light source to apply laser light energy at approximately about 970nm to a target tissue and disrupt sweat glands in the human body. It is to be understood that the use of the term "about" in the present application means a variance of +/- 10. Further, the controller controls the laser light energy to be delivered pulsed. The controller can also control a cooling system and/or a vacuum system so that a target skin area may be drawn into the handpiece prior the application of the light energy and cooled before, during or after the application of the light energy.

In the method above and the apparatus above, the application of laser light energy has the following parameters: (a) fluence of 5-50 j/cm2; (b) pulse width of 10- 60ms; (c) number of pulses of 1-20; (d) repetition rate of 0.3 - 5Hz; (e) laser wavelength of about 970nm

### BRIEF DESCRIPTION OF THE DRAWINGS

Figure 1 illustrates skin surface tissue and the relation of sweat glands to the skin surface tissue.
Figure 2 illustrates a typical physiology of the relation of sweat glands within an area of fat cells.
Figure 3 illustrates a number of spectral absorption characteristics of tissue components at various wavelengths of light.
Figures 4A and 4B illustrate the results of treatments using the apparatus and methods of the present invention on sweat glands.
Figure 5 illustrates an exemplary handpiece that may be used with the method and apparatus of the present invention.
Figures 6A and 6B illustrate an example of the pulse selective profile of temperature differential effects on fat tissue vs. water soluble tissue components when a 1 Hz pulse is applied to the skin tissue, using wavelengths of 1060nm and 970nm respectively.

### DETAILED DESCRIPTION OF THE PRESENT INVENTION

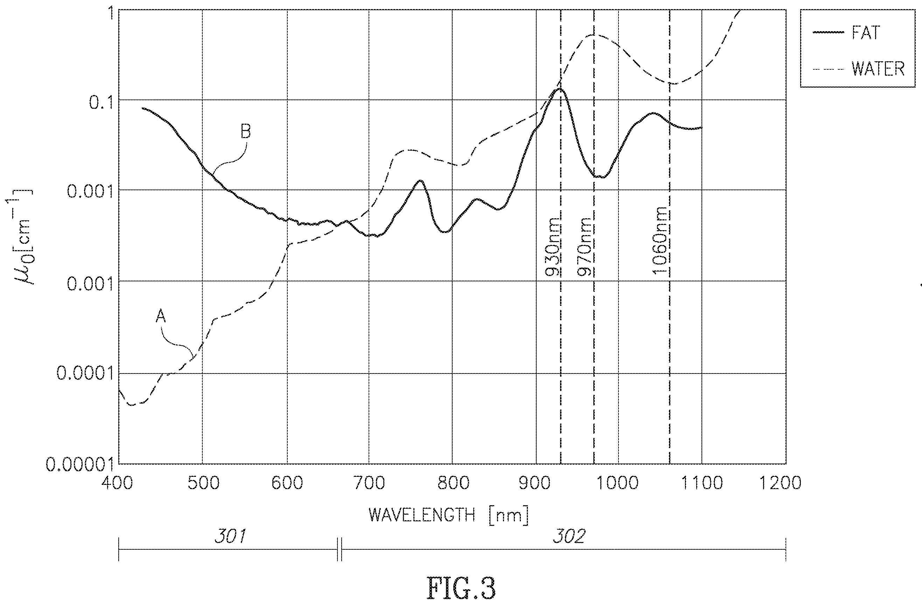

In the present invention, a non-invasive treatment of hyperhidrosis is accomplished by means of an external laser source. The objective is to cause selective thermally induced necrosis and/or apoptosis of sweat gland while maintain the surrounding tissue intact. As mentioned above, there are several methods to obtain that objective. The key element to proper selective treatment is the relative absorption of electromagnetic radiation by the various tissue components. Figure 3 presents plots A and B of the spectral behavior of light absorption for fat and water respectively. As can be seen in Fig. 3, around wavelengths 650nm - 660nm there is an isosbestic point 300 between fat and water. In region 301, at wavelengths shorter than that of the isosbestic wavelengths, fat absorption is higher than water absorption. In region 302, at wavelengths longer than that of the isosbestic wavelengths, water absorption is higher than fat absorption. As will be explained in more detail below, according to one aspect of the present invention, selective sweat gland treatment in region 301 may be achieved by a bulk heating treatment mode.

Tissue bulk heating may be achieved by using a pulsed or a continuous wave laser. In this treatment mode, the entire bulk of the tissue will be heated homogenously to approximately the same temperature while treatment selectivity may be achieved due to the differences in the Damaging Temperature Threshold (DTT) which characterizes sweat glands versus the surrounding fat. Since the sweat glands' DTT is lower than that of fat, selectivity may be achieved by increasing the bulk temperature to a level which is higher than the DTT for sweat glands but lower than the DTT of fat.

By using the radiation wavelengths in region 301, the sweat glands which are surrounded by fat tissue by heating the surrounding fat layer are targeted. Thus, the fat tissue can be targeted and its temperature increased to a certain level that initiates necrotic and/or apoptotic processes in the sweat glands embedded in it, while leaving the dermis tissue at a lower temperature.

Irradiating the tissue with wavelengths in the region 301 of Figure 3 will raise the temperature of fat faster than that of the sweat glands. However, since the sweat glands are encapsulated by fat tissue, heat will diffuse from that tissue to the gland and increase its temperature. By adopting an appropriate irradiation protocol, the bulk temperature may be elevated to a point which is above the sweat gland DTT but below the fat cells' DTT, leading to a selective treatment of sweat glands.

A waveband which favors absorption by fat compared to water, such as 400nm - 650nm, may be used. In this case, low power continuous wave (CW) or continuously oscillating (or pulsed) radiation having relatively small peak power and substantially high repetition rate may be applied to the treated region. Gliding a laser beam producing head back and forth over a wide region in order to gradually and homogenously increase the targeted area temperature and maintain it certain level is commonly known and may be applied to treat the sweat glands as well. As before, applying cooling to the epidermal layer reduces the risk of adverse effects.

In contrast, in region 302, the sweat glands are targeted directly to increase their temperature to sufficient levels and for a sufficient duration. Since, in this part of the spectrum, the absorption of light in water is higher than is fat, the sweat glands' temperature will increase faster than in the surrounding fat layer. This enables a selective treatment which is based on non-homogenous heating of the target tissue and direct targeting of the sweat glands. In addition, the fatty layer which surrounds the water based sweat gland thermally isolates it from the environment and effectively increases its thermal relaxation time (TRT). Thermal relaxation time is the duration required for the heat generated by absorbed light energy within the targeted chromophore to dissipate to 50% of its value immediately after laser exposure. A longer TRT enhances the selectivity of the treatment as the sweat gland will maintain its elevated temperature for a longer time, increasing the probability of initiation of apoptotic and/or necrotic processes.

Based on the facts outlined above, when selecting the specific wavelength within region 302 with which to treat, the desire may be to use a wavelength that maximizes the absorption difference between fat and water. On the other hand, the desire is for the absorption in water not to be too high. This could result in high absorption of the radiation by the epidermis and upper dermis layers (small penetration depth), with very little energy penetrating to the deeper levels of the dermis, and not be enough to affect the sweat glands.

It appears that the optimal wavelength for such a treatment may be in the 930nm-1030nm band, preferably at approximately 970nm. Therefore, according to an embodiment, one suggested procedure for treating the sweat glands is with a diode laser at approximately 970nm, based on the above analysis. The absorption at these wavelengths of energy in water is preferential compared to their absorption in fat. This is illustrated in Fig.3A which shows that 970nm produces a greater differential absorption between fat and water (sweat gland contents) when compared, for example, to 930nm or 1060nm. 970nm is capable of penetrating deep into the hypodermal layer and affect the water-soluble components on the way, such as the sweat glands. Due to the fact that, as mentioned above, the sweat glands are surrounded by thermal isolation tissue (in this case fat), which has lower absorption properties at this wavelength, relatively selective necrosis of the sweat glands can be achieved.

Thus, using approximately 970nm may be seen as selectively targeting the sweat glands themselves over the surrounding tissue. However, as will be discussed below, differential absorption is not a panacea in and of itself, such that even lesser differentials may be useful in damaging sweat glands due to the morphological structure of water from the secretory cells embedded in fat layer, from, for example, bulk heating.

Another optional waveband in region 302 is 1030nm - 1200nm, although it may be expected to have lower efficacy than 970nm. However, turning now to Figs. 6A and 6B, it can be seen that at 1060nm, while the absorption at that wavelength in fat tissue is greater than that shown for 970nm, it is not overly high and the absorption in the sweat gland is equivalent to that shown for 970nm so that selectivity may also be achieved in this portion of the spectrum.

The 680nm - 910nm waveband is another band, in region 301, that may be applied to sweat gland treatments, but that range may be expected to be more effective in the bulk heating of skin tissue as discussed herein

By providing selective heating of fat and the sweat glands, necrotic processes may be induced only in the sweat gland while leaving the surrounding tissue intact. Various pulse sequences using the parameters listed in table 1 were tested with a laser having a wavelength of 970nm and indeed resulted in very selective damage to the sweat glands.

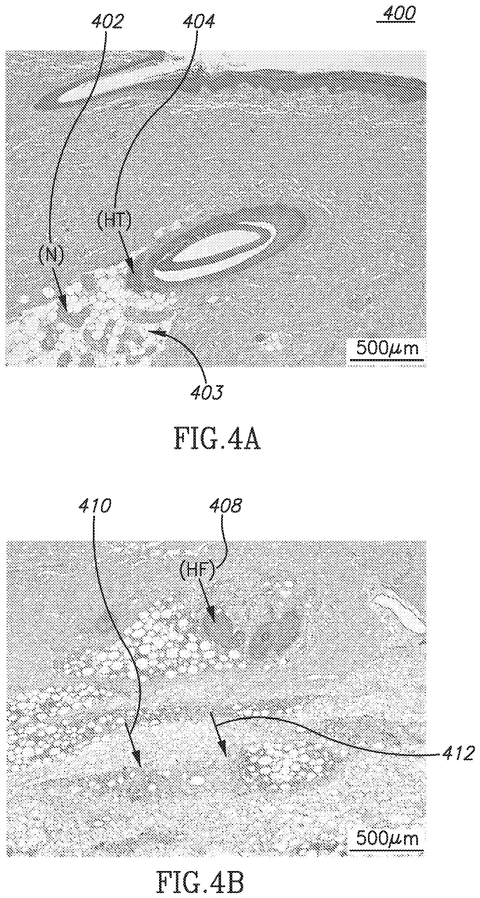

In the drawing sequence 400 of Figs. 4A and 4B, Fig. 4A generally illustrates the effects of such a pulse sequence on a pig's skin one week after treatment and Figure 4B one month after the treatment. Turning now to Fig. 4A, that figure shows a hair follicle in the central portion of the photograph of Fig. 4A with a hemorrhagic wound 404 (HT). Fig. 4A also shows necrotic dark-colored (in the photograph) sweat gland cells 402 (N) surrounded by lighter colored fat cells 403(in the photograph). Turning now to the photograph of Fig. 4B, one month later this figure shows a hair follicle 408 (HF), surrounded by subchronic inflammation (shown by arrows 410 and 412) but notably that there remains no remnant of the sweat gland components in areas in which they were previously present. Thus, this sequence 400 of Figs. 4A and 4B illustrates the selective inflammatory effect in the sweat gland can be observed (indicating a necrotic process) after one week, and the disappearance of the sweat gland after a month.

**Table 1 above provides an exemplary set of pulse sequences characteristics which may be used to treat hyperhidrosis**

| Parameter | Value |
|---|---|
| Fluence [j/cm2]: | 5 to 50 |
| Pulse widths [ms]; | 10 to 60 |
| Number of pulses: | 1 to 20 |
| Repetition rates [Hz]: | 0.3 to 5 |

In order to reduce the risk of adverse effects such as burns on the epidermis to radiation absorption by melanin, local cooling may be employed during treatment. Such cooling may be accomplished using, for example, the built-in cooling mechanism provided by the "chilled tip" technology available in some products made by Lumenis Ltd. of Israel in its LIGHTSHEER^{®} and RESURFX^{®} product lines. Other cooling methods can be considered as well. Pre- and post-cooling further reduce side effects such as edema or pain, may provide partial anesthesia, and may reduce temperature-rise in the melanin-rich epidermis.

Cooling may be provided continuously or intermittently and may be applied during the treatment or in a combination of during the treatment and post treatment to minimize undesired skin response such as erythema, edema, purpura, without interfering with the mechanism of heat accumulation in the sweat glands. Topical cooling of skin surface during or after energy delivery to the tissue may avoid heat accumulation even in upper layer of dermis, contributing to the procedure safety and allowing more energy to be transmitted to the tissue as and if needed.

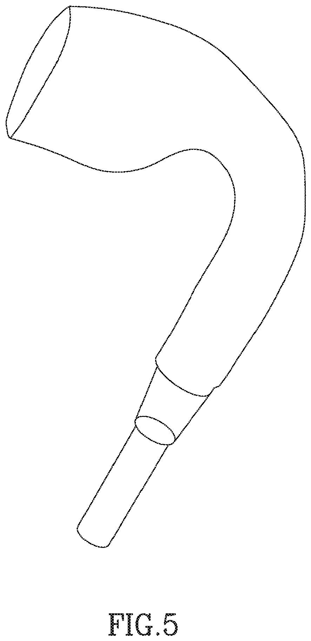

Further enhancement of treatment efficacy might be obtained when delivering the treatment by applying vacuum to the treated area. The handpiece illustrated in Figure 5 is a device to deliver laser light energy; the handpiece includes an open tip and a source of vacuum, and the process of using it includes a step of drawing the skin tissue into the open tip prior to applying the laser light energy, such that the sweat glands may be drawn closer to the laser light source contained within the handpiece. The handpiece of Figure 5 is described in US 2008/0119830, assigned to the same assignee of the present invention or may be the Lightsheer^{®} HS laser device also made by the same assignee. An additional advantage of applying vacuum to the tissue may be vaso-constriction of the blood vessels, thus pushing the blood from the target area and further enhancing the absorption of the light energy from the laser energy source into the sweat glands rather than being absorbed in competitive chromophores.

While the foregoing has discussed wavelengths in the regions 301 for bulk heating and in region 302 for more targeted heating, the wavelengths do not need to be restricted to either one of the wavelengths or spectrums mentioned above, but may also be a combination of a "mix and match" of two or more of the above wavelengths or spectrums, from the same region 301 or 302 or from both regions, according to the planned treatment of affecting the sweat glands and/or the fatty components. Also, a combination of bulk heating and, as appropriate, pulsed treatments may be combined to achieve the desired effects of damaging the sweat glands. A programmed controller may be employed to control and sequence these different treatment parameters.

While the foregoing general discussion has been directed to treatment of axillar sweat glands, it is to be understood that other body parts may be treated with the present inventions, such as the soles, palms, forehead, back, chest, and groin, as well as in the treatment of bromhidrosis (foot odor) and osmidrosis (heavy sweat odor) in various parts of the human body.

Typically, for a chilled tip hand piece configuration, the hand piece will be positioned using a "pick and place" or "gliding" or "painting", depending on the wave form used, while maintaining firm compression during the entire treatment.

In the "pick and place" method, the tip is picked up from the skin immediately after the pulse, moved to the next treatment location, and then lowered against the skin. In the "gliding" technique, the tip is kept continuously in contact with the skin and moved to the next treatment location immediately after the laser pulse, making sure that the sapphire tip is pressed to - or in close contact with - the skin when the laser light is applied. In the "painting" technique, the operator may move the handpiece back and forth over the skin surface.

## Claims

1. Apparatus for treating a human body to reduce hyperhidrosis comprising:
a source of laser light energy; and
a controller for controlling the laser light source wherein the controller causes the laser light source to apply laser light energy at 970nm plus or minus 10nm to target and disrupt sweat glands (204) in the human body, wherein the controller controls the laser light energy to be pulsed and wherein the controller controls the laser light source to operate at the following parameters
(a) fluence of 5-50 j/cm2;
(b) pulse width of 10-60ms;
(c) number of pulses of 1-20;
(d) repetition rate of 0.3 - 5Hz.

2. A cosmetic method of treating a human body to reduce hyperhidrosis comprising:
applying laser light energy to one or more portions of the human body wherein the laser light energy has a wavelength between 970nm plus or minus 10nm and controlling the application of laser light to target and disrupt sweat glands (204) in the human body directly by increasing the temperature to sufficient levels and for a sufficient time inducing necrosis and/or apoptosis only in the sweat glands (204) while leaving the surrounding tissue intact, by controlling the application of laser light at the following parameters:
(a) fluence of 5-50 j/cm2;
(b) pulse width of 10-60ms;
(c) number of pulses of 1-20;
(d) repetition rate of 0.3 - 5Hz.

3. The cosmetic method of claim 2, wherein the pulse rate is 1 Hz.

4. The cosmetic method of claim 2, wherein the pulse rate is selected from between ½ Hz and 3 Hz.

5. The cosmetic method of claim 2, further comprising the step of cooling the human body in the vicinity of the application of the laser light energy.

6. The cosmetic method of claim 2, wherein the time delay between pulses is between the thermal relaxation time, TRT, of fat cells and the TRT of sweat glands.

7. The cosmetic method of claim 2 further comprising the step of providing bulk heating of the skin surface of the human body one or more of: before, during and after the application of the laser light energy.

8. The cosmetic method of claim 7, wherein the bulk heating is provided by one or more of: laser light energy, IPL light energy, RF energy, microwave energy or ultrasonic energy.

9. The cosmetic method of either claim 7 or 8, wherein the bulk heating is applied at about 400 to about 650nm.

10. The cosmetic method of claim 2, further comprising providing a handpiece to deliver the laser light energy and wherein the handpiece includes an open tip and a source of vacuum, and further comprising the step of drawing the skin tissue into the open tip prior to applying the laser light energy.

## Patentansprüche

1. Vorrichtung zur Behandlung eines menschlichen Körpers zur Reduzierung von Hyperhidrose, umfassend:
eine Quelle für Laserlichtenergie; und
eine Steuerung zum Steuern der Laserlichtquelle, wobei die Steuerung bewirkt, dass die Laserlichtquelle veranlasst, Laserlichtenergie bei 970nm plus oder minus 10nm anzuwenden um die Schweißdrüsen (204) im menschlichen Körper anzuvisieren und zu schädigen, wobei die Steuerung die Laserlichtenergie so steuert, dass sie gepulst wird, und wobei die Steuerung die Laserlichtquelle so steuert, dass sie mit den folgenden Parametern arbeitet
(a) Fluenz von 5-50 J/cm2;
(b) Pulsbreite von 10-60ms;
(c) Anzahl der Pulse von 1-20;
(d) Wiederholungsrate von 0,3 - 5 Hz.

2. Kosmetisches Verfahren zur Behandlung eines menschlichen Körpers zur Verringerung von Hyperhidrosis umfassend:
Anwendung von Laserlichtenergie auf einen oder mehrere Bereiche des menschlichen Körpers, wobei die Laserlichtenergie eine Wellenlänge zwischen 970nm plus oder minus 10nm hat und Steuern der Anwendung von Laserlicht, um die Schweißdrüsen (204) im menschlichen Körper anzuvisieren und zu schädigen durch Erhöhen der Temperatur auf ausreichende Werte und für eine ausreichende Zeit, um Nekrose und/oder Apoptose nur in den Schweißdrüsen (204) zu induzieren, während das umgebende Gewebe intakt bleibt, wobei das Verfahren die Anwendung von Laserlicht mit den folgenden Parametern umfasst:
(a) Fluenz von 5-50 J/cm2;
(b) Pulsbreite von 10-60ms;
(c) Anzahl der Pulse von 1-20;
(d) Wiederholungsrate von 0,3 - 5 Hz.

3. Kosmetisches Verfahren nach Anspruch 2, wobei die Pulsrate 1 Hz beträgt.

4. Kosmetisches Verfahren nach Anspruch 2, wobei die Pulsrate ausgewählt ist zwischen ½ Hz und 3 Hz.

5. Kosmetisches Verfahren nach Anspruch 2 umfassend ferner den Schritt des Kühlens des menschlichen Körpers in der Nähe der Anwendung der Laserlichtenergie.

6. Kosmetisches Verfahren nach Anspruch 2, wobei die Zeitverzögerung zwischen den Pulsen zwischen der thermischen Relaxationszeit TRT von Tat-Zellen und der TRT von Schweißdrüsen liegt.

7. Kosmetisches Verfahren nach Anspruch 2 umfassend ferner den Schritt der Bereitstellung einer Massenerwärmung der Hautoberfläche des menschlichen Körpers, und zwar vor, während und nach der Anwendung der Laserlichtenergie.

8. Kosmetisches Verfahren nach Anspruch 7, wobei die Massenerwärmung durch eines oder mehrere der folgenden Mittel bereitgestellt wird: Laserlichtenergie, IPL-Lichtenergie, RF-Energie, Mikrowellenenergie oder Ultraschallenergie.

9. Kosmetisches Verfahren nach Anspruch 7 oder 8, wobei die Massenerwärmung bei etwa 400 bis etwa 650nm erfolgt.

10. Kosmetisches Verfahren nach Anspruch 2 umfassend ferner das Bereitstellen eines Handstücks zum Liefern der Laserlichtenergie, wobei das Handstück eine offene Spitze und eine Vakuumquelle aufweist, und umfassend ferner den Schritt des Ansaugens des Hautgewebes in die offene Spitze, bevor die Laserlichtenergie angewendet wird.

## Revendications

1. Appareil de traitement d'un corps humain pour réduire l'hyperhidrose comprenant :
une source d'énergie lumineuse laser ; et
un organe de commande pour commander la source lumineuse laser dans lequel l'organe de commande amène la source lumineuse laser à appliquer une énergie lumineuse laser à 970 nm plus ou moins 10 nm pour cibler et perturber les glandes sudoripares (204) dans le corps humain, dans lequel l'organe de commande commande l'impulsion de l'énergie lumineuse laser et dans lequel l'organe de commande commande le fonctionnement de la source lumineuse laser selon les paramètres suivants
(a) fluence de 5 à 50 j/cm² ;
(b) largeur d'impulsion de 10 à 60 ms ;
(c) nombre d'impulsions de 1 à 20 ;
(d) taux de répétition de 0,3 à 5 Hz.

2. Procédé cosmétique de traitement d'un corps humain pour réduire l'hyperhidrose comprenant :
l'application d'une énergie lumineuse laser à une ou plusieurs parties du corps humain dans lequel l'énergie lumineuse laser a une longueur d'onde de 970 nm plus ou moins 10 nm et la commande de l'application de lumière laser pour cibler et perturber les glandes sudoripares (204) dans le corps humain directement par augmentation de la température à des niveaux suffisants et pendant une durée suffisante induisant une nécrose et/ou une apoptose uniquement dans les glandes sudoripares (204) tout en laissant le tissu environnant intact, en commandant l'application de lumière laser aux paramètres suivants :
(a) fluence de 5 à 50 j/cm² ;
(b) largeur d'impulsion de 10 à 60 ms ;
(c) nombre d'impulsions de 1 à 20 ;
(d) taux de répétition de 0,3 à 5 Hz.

3. Procédé cosmétique selon la revendication 2, dans lequel le taux d'impulsions est de 1 Hz.

4. Procédé cosmétique selon la revendication 2, dans lequel le taux d'impulsions est sélectionné entre 1/2 Hz et 3 Hz.

5. Procédé cosmétique selon la revendication 2, comprenant en outre l'étape de refroidissement du corps humain à proximité de l'application de l'énergie lumineuse laser.

6. Procédé selon la revendication 2, dans lequel le délai temporel entre les impulsions est entre le temps de relaxation thermique, TRT, des cellules adipeuses et le TRT des glandes sudoripares.

7. Procédé cosmétique selon la revendication 2 comprenant en outre l'étape de fourniture d'un chauffage global de la surface cutanée du corps humain un ou plusieurs de : avant, pendant et après l'application de l'énergie lumineuse laser.

8. Procédé cosmétique selon la revendication 7, dans lequel le chauffage global est fourni par un ou plusieurs de : une énergie lumineuse laser, une énergie lumineuse IPL, une énergie RF, une énergie micro-ondes ou une énergie ultrasonore.

9. Procédé cosmétique selon l'une ou l'autre de la revendication 7 ou 8, dans lequel le chauffage en masse est appliqué à environ 400 à environ 650 nm.

10. Procédé cosmétique selon la revendication 2, comprenant en outre la fourniture d'une pièce à main pour délivrer l'énergie lumineuse laser et dans lequel la pièce à main inclut une pointe ouverte et une source de vide, et comprenant en outre l'étape d'aspiration du tissu cutané dans la pointe ouverte avant l'application de l'énergie lumineuse laser.
